# EUROPEAN PATENT APPLICATION

(11) **EP 1 702 642 A1**
(43) Date of publication of application: **20.09.2006**
(21) Application number: 05251620.0
(22) Date of filing: 17.03.2005
(51) Int. Cl.: A61M 25/02

(54) **A device for fixating a catheter to a skin**

(71) Applicant: Unomedical Limited, Redditch, Worcestershire B98 9N1 (GB)
(72) Inventor: Cummings, Edward Andrew, Armagh Co. Armagh BT61 9EP (GB); Southwell, James Edward, Brockworth Gloucester GL3 4RH (GB)
(74) Representative: Nielsen, Henrik Sten

(57) **Abstract**

A fixation device (10) for fixating a catheter relative to a skin comprises a basic foil (12) having opposite first and second surfaces and defining a first end. A first adhesive coating is applied to the first surface. A first pad (24) is included in the device intended to be arranged adjacent to a perforation site, defining a convex marginal surface having a radius of curvature of at least one order of magnitude larger than the outer diameter of a catheter (32). The marginal surface provides a support surface for the catheter positioned in facial contact therewith for arranging the catheter in a loop and turning the catheter from an orientation substantially perpendicularly relative to the skin to an orientation substantially parallel with the skin and for preventing the catheter from sliding along the marginal surface. The first pad is supported by the basic foil at the first surface of the basic foil. A second adhesive coating is applied to a part of the second surface of the basic foil below the first pad and defining a rectilinear adhesive material boundary line constituting a folding line (17) for folding the basic foil onto the first pad.

## Description

The present invention relates to a device for fixating an epidural catheter or a similar drainage catheter to a skin surface part of a patient or person.

The present invention generally relates to the technique of applying and fixating a catheter, such as an epidural catheter, relative to a skin surface part of a patient or person, and more precisely a device for fixating such a catheter relative to a skin surface part of a patient or person.

It is a well-known technique to enter a catheter through the skin of a patient or person, e.g. after a surgical operation.

In certain applications, tiny catheters are to be used which have to be applied to e.g. the back of the person or patient The size of the catheter itself makes the catheter extremely susceptible to blockings in case the catheter is not properly mounted and in case the patient or person lies on the catheter. The small size of the catheter, e.g. of an epidural catheter also renders it highly likely that the catheter is withdrawn from its intentional position as the patient or person shifts his or her position sitting or lying in the bed or is moved to and from a bed, a chair or moves around as the catheter is very easily caught by the patient or person or caught by a person helping the patient or person.

Furthermore, the technique of applying and fixating a catheter, in particular an epidural catheter, is a complex and time-consuming operation which requires skill and which further often turns out to be inadequate and inappropriate as the epidural catheter is easily pulled out and removed from its intentional position.

Various devices for fixating a catheter have recently been developed as disclosed in eg. GB patent No. 2,115,290, US patent No. 4,419,094, US patent No. 4,645,492, US patent No. 5,282,791, US patent No. 5,370,627, US patent No. 5,372,589, WO 91/07204, WO 91/17738, WO 95/33508, WO 93/25264 and WO 93/17738- Reference is made to the above patents and patent applications, and the above US patents are hereby incorporated in the present specification by reference.

The above-mentioned U.S. Patent No. 5,372,589 discloses a transparent self-adhesive film dressing with a special aerating fenestra, which substantially covers and secures an intravenous catheter to the skin of the patient, for visual inspection, without encouraging growth of bacteria. Although the above-mentioned film dressing is advantageous from the point of view of its extremely simple construction, it can only be used in connection with catheters for percutaneous intubations, which catheters are introduced practically tangentially to the patient's skin, and not in connection with catheters which are introduced perpendicularly to the patient's skin, such as epidural catheters. Application of the self-adhesive film dressing according to U.S. Patent No. 5,372,589 in connection with an epidural catheter would cause the epidural catheter to bend brusquely at the exit from the puncture point, provoking thus a constriction of the catheter flow channel and impeding an uninterrupted epidural collection and would not prevent the epidural catheter from being pulled out from the position.

The above mentioned published international patent application, publication No. WO 93/25264 discloses a device for fixating a drainage tube, and a drainage tube assembly introduced perpendicularly to the patient's skin, which device comprises a support component including a flange part and a tubular part which are integrally connected through a conical part. The tubular part has a through-going passage for receiving the drainage tube, and the flange part has a surface part to be arranged in surface contact with the skin surface part of the patient. The device also comprises a locking component for locking the drainage tube relative to the tubular part of the support component. The particular assembly of the flange part, the conical part and the tubular part eliminates the risk that the drainage tube may bend at the entrance of the patient's skin and the risk of creation of kinks in the drainage tube. However, the device according. to publication No. WO 93/25264 has appreciable dimensions in all three coordinates, making it uncomfortable for the patient if he/she will make a move and squeeze the tube assembly between his/her skin and the bed, at the same time provoking also the interruption of flow in the tube or even the breaking thereof.

From EP 1 007 133, a device for fixating an epidural catheter or similar catheter relative to a skin surface part of a patient or person is known, which device to a high degree solves the problems inherent to the devices known from the above listed patents and patent appliactions.

It has, however, been realised that the fixation device known from the above European patent may be further improved or refined for allowing an even more easy and fail safe mounting of the device and fixation of the epidural catheter or similar catheter relative to the skin surface part of the patient or person.

An object of the present invention is to provide a device for fixating an epidural catheter or similar catheter, which device eliminates the risk that the catheter may bend abruptly at the perforation site in the patient's skin or create kinks, which prevents the catheter from being pulled out in case the catheter is unintentionally squeezed, pressed or in any other way removed from a resting position, and which inherently ensures that the device is always properly and correctly mounted as the device includes a fail safe blocking preventing inacurate or incorrect mounting of the device.

A particular advantage of the device according to the present invention resides in that a single unitary structure is provided which renders it possible, in a single and easily performable operation, to accomplish a secure fixation of the epidural catheter and also make the catheter arid the catheter flow less sensitive to the patient's moves or to external pull, squeeze, press or jerk factors, and which ensures or guarantees that the secure fixation of the epidural catheter is always carried out in the intentional and correct position.

A particular feature of the device according to the present invention relates to the face that the device is made from a minimum number of components, namely a basic foil and one or more supporting pads, which foil is provided with skin friendly adhesive coatings ad dedicated or specific areas of the basic foil.

A further particular feature of the device according to the present invention lies in that the device according to the present invention constitutes a disposable unitary structure which has been presterilized and is applied in a simple manner as a plaster structure to the patient's skin:
The above object, the above advantage and the above features together with numerous other objects, advantages and features which Will be evident from the description below are obtained by means of a fixation device for fixating a catheter relative to a skin surface part of a person, said catheter defining an outer diameter and being introduced into the interior of said person through said skin surface part substantially perpendicularly relative thereto through a perforation site, comprising:
a basic foil of a foil material having opposite first and second surfaces and defining a first end,
a first adhesive coating applied to said first surface of said basic foil,
a first pad to be arranged adjacent to said perforation site and defining a convex marginal surface having a radius of curvature of at least one order of magnitude larger than said outer diameter of said catheter, said marginal surface providing a support surface in relation to said catheter positioned in facial contact therewith for arranging said catheter in a loop and turning said catheter from an orientation substantially perpendicularly relative to said skin surface part to an orientation substantially parallelly with said skin surface part and for preventing said catheter from sliding along said marginal surface, said first pad being connected to and supported by said basic foil at said first surface of said basic foil, and
a second adhesive coating applied to a part of said second surface of said basic foil below said first pad and defining a rectilinear adhesive material boundary line constituting a folding line for folding said basic foil onto said first pad in a specific position relative thereto and covering said first pad, and for arranging said first surface of said basic foil facing towards said first pad for adhering to said skin surface part, for fixating said catheter relative to said first pad and for covering said perforation site.

In accordance with the basic realisation characteristic of the present invention, the second adhesive coating applied to a part of the second surface of the basic foil serves, in the first place, to fixate the first pad in its intentional position adjacent to the perforation site and also, and characteristic of the present invention, of providing a specific generator for the folding of the basic foil onto itself and onto the epidural catheter for fixating and covering the epidural catheter and also the perforation site by the presence of the rectilinear adhesive material boundary line at which the folding is initiated.

The first pad of the fixation device according to the present invention constitutes an element which serves the dual purpose of turning the catheter from an orientation substantially perpendicularly relative to the skin surface part to an orientation substantially parallelly with the skin surface part and supporting the catheter in a loop preventing the catheter from sliding along the marginal surface defined by the first pad and consequently preventing the catheter from being pulled out or otherwise removed from its intentional position extending through the skin of the person through the perforation site.

According to a particular advantageous embodiment of the fixation device according to the present invention, a second pad is provided, which second pad is positioned adjacent to the first pad for defining with the first pad a constriction having a width slightly smaller than, such as constituting 50-50%, e.g. 60-80% of the outer diameter of the catheter for arresting the catheter within the constriction, when the catheter is received therein.

According to a further refinement of the above described presently preferred and advantageous embodiment of the fixation device according to the present invention, the constriction constitutes a tapering constriction for allowing the catheter to slide relative to slide relative to the constriction in the one direction relative to the constriction and preventing the catheter from sliding in the opposite direction, thereby allowing the catheter to be easily positioned correctly by sliding the catheter relative to the constriction in the first mentioned direction, whereupon any unintentional movement such as pulling the catheter in the opposite direction after the proper fixation of the catheter is prevented.

The fixation device according to the present invention basically comprises two main components viz the first pad serving the above described dual purpose and the basic foil.

The terms foil and sheet are in the present context to be considered synonymous and are to be considered generic expressions or terms defining a geometric element which has opposite surfaces of fairly large dimensions as compared to the overall thickness of the element in question. The terms foil and sheet are by no means to be construed limited to specific materials and interpretations in relation to e.g. plastic materials, however are to be understood covering plastic foils or sheets and equivalent elements serving the same purpose as plastic foils or sheets.

For further improving the fixation of the catheter relative to the skin surface part of the patient or person, the basic foil according to the above described preferred embodiment of the device according to the present invention preferably has an extension serving the purpose of covering and adhering to the catheter.

Dependent on the configuration of the first pad and also the configuration of the basic foil, the first pad may be provided with an adhesive covering at the convex marginal surface for causing the catheter in the above described loop to adhere to the first pad. Additionally or alternatively, the first pad may be provided with an adhesive surface covering for adhering to the basic foil as the basic foil is folded onto the first pad.

The device according to the present invention may comprise an extension of the basic foil for covering and adhering to the catheter for improving the fixation of the catheter relative to the skin surface part. Furthermore or alternatively, the basic foil may be provided with a fixation support adjacent the first pad for attaching the catheter to the skin surface part of the patient or person.

For improving the purpose of preventing that the catheter is bent abruptly or blocked, the first pad may be provided with one or more notches for receiving and fixating the catheter relative to the first pad and consequently supporting and properly securing the catheter relative to the first pad.

In order to allow visual inspection of the perforation site and also allow visual inspection of the arrangement of the catheter relative to the device according to the present invention and consequently relative to the perforation site, the basic foil may advantageously be provided with a transparent area allowing visual inspection of the perforation site.

In order to render it possible to handle the fixation device before applying it to the catheter and to the skin surface part of the person, to which skin surface part the catheter is to be fixated, the device according to the present invention preferably further comprises release foils covering all the adhesive areas. The release foils may be constituted by a siliconized paper, siliconized or non-siliconized polyester, polypropylene, polyethylene or a similar plastic material.

The basic foil of the fixating device according to the present invention may be configurated in an arbitrary shape provided the basic foil fulfils the above requirement of fixating the catheter and the catheter-supporting basic foil relative to the skin surface part. Thus, according to alternative geometrical embodiments of the fixation device according to the present invention, the basic foil may be of a rectangular configuration, a quadratic configuration, a circular configuration, an elliptic configuration or a combination of the above configurations.

For preventing the ingress of bacteria into the perforation site, the foil covering the perforation site is preferably made from a material providing a bacteria barrier. Provided the basic foil as discussed above is provided with a transparent area, the transparent area is advantageously and preferably covered by a gas and liquid impermeable and bacteria barrier defining transparent foil. Alternatively, provided the basic foil is provided without a transparent area, the basic foil or at least a part of the basic foil covering the perforation site is preferably constituted by a material providing a bacteria barrier.

The sheet material of the basic foil of the fixation device according to the present invention may be made from any appropriate material which may be used as a basic foil material. Provided the adhesive layers are substantially water-impermeable, the sheet material may be made from water-impermeable or water-permeable material. in order to hinder any penetration of water through the basic foil from the environment into the area covered by the bandage, the sheet material is, however, preferably a substantially water-impermeable material such as spun bond, spun lace, woven or non-woven foil materials of polyester, nylon, polypropylene, polyethylene, polyurethane, polyvinylchloride, clear tape or other transparent or non-transparent material, or combinations thereof.

The pad or pads may be made of any appropriate soft material such as a fibrous synthetic or natural material, e.g. cotton, viscose, rayon or the like, synthetic or natural rubber or foamed plastic foils such as a polyester foam, a polyurethane foam, EVA foam (Ethylene Vinyl Acetate foam).

The individual components of the fixation device according to the present invention including the basic foil, the adhesive covering and also any additional adhesives and/or release foils etc. may readily be chosen by a person having ordinary skill in the art from materials which are well-known in the art for medical application including medical grade supporting element materials, basic foils, release foils, adhesives etc.

The present invention also relates to a catheter system comprising a catheter, in particular an epidural catheter and also a fixation device according to the present invention.

The adhesive layer may be constituted by a medical grade acrylic adhesive, a medical, natural or synthetic rubber resin adhesive or other medical grade adhesive.

The invention will now be further described with reference to the drawings, in which:

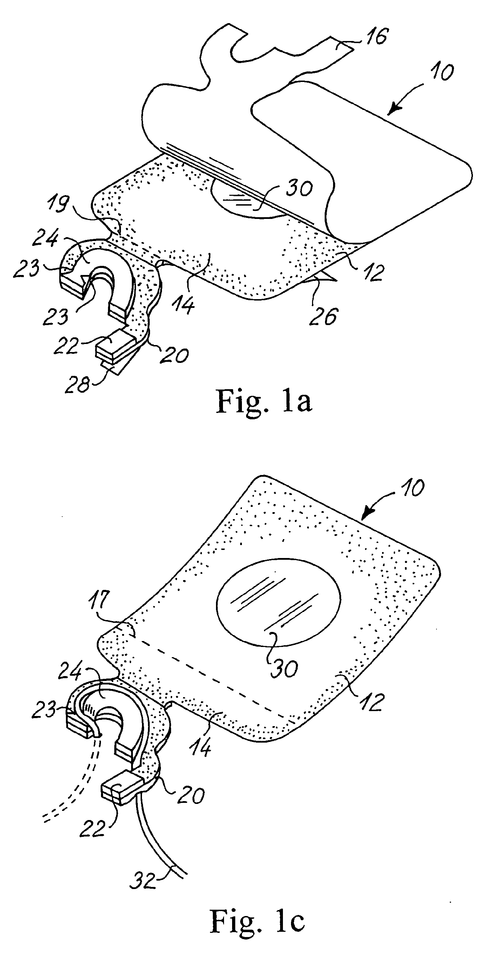

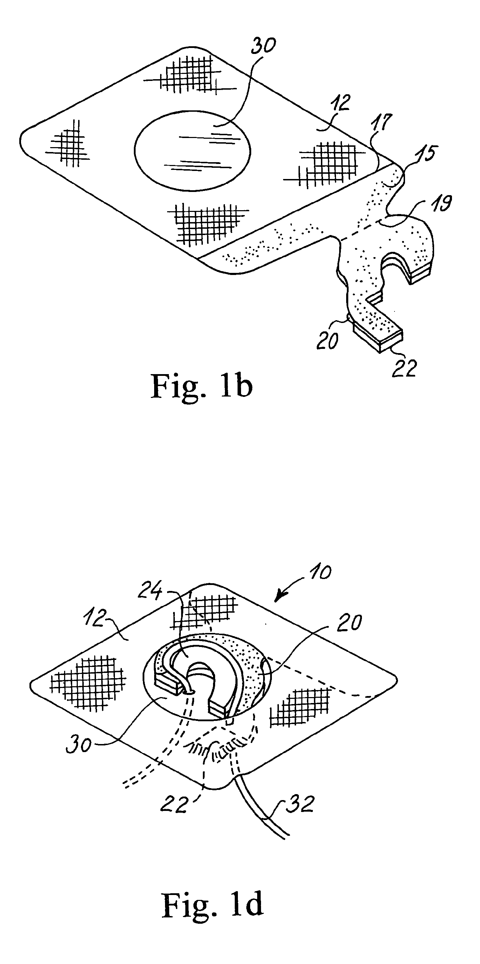

Fig. 1 a is a schematic and perspective top view of a first embodiment of an epidural catheter fixation device according to the present invention,
Fig. 1 b is a schematic and perspective bottom view of the epidural catheter fixation device of Fig. 1a,
Fig. 1c is a schematic and perspective top view of the embodiment of the epidural catheter fixation device of Figs. 1a and 1b, illustrating a first step of applying the epidural catheter fixation device for fixating an epidural catheter relative to a skin surface part of a person or patient,
Fig. 1 d is a schematic and perspective view illustrating the epidural catheter of Fig. 1 c finally fixated by means of the epidural catheter fixation device,
Figs. 2a and 2b are schematic and perspective top and bottom views, respectively, similar to the views of Figs. 1 a and 1 b, respectively, of a second embodiment of the epidural catheter fixation device according to the present invention,
Figs. 3a and 3b are schematic and perspective top and bottom views, respectively, similar to the views of Figs. 1a and 1b, respectively, of a third embodiment of the epidural catheter fixation device according to the present invention,
Figs. 4a and 4b are schematic and perspective top and bottom views, respectively, similar to the views of Figs. 1a and 1b, respectively, of a fourth embodiment of the epidural catheter fixation device according to the present invention.
Fig. 4c is a schematic and perspective view similar to the view of Fig. 1d of the fourth embodiment of the epidural catheter fixation device,
Figs. 5a and 5b are schematic and perspective top and bottom views, respectively, similar to the views of Figs. 1 a and 1b, respectively, of a fifth embodiment of the epidural catheter fixation device according to the present invention, and
Fig. 5c is a schematic and perspective top view of the fifth embodiment of the epidural catheter fixation device according to the present invention disclosing the device after the removal of release papers.

In the following examples, parts and elements of different embodiments of an epidural catheter fixation device having the same function are designated the same reference numeral but primed a number of times, the priming number being one unit less than the number of the embodiment. For example, an element 20 in the first embodiment will be designated the reference numeral 20' in the second embodiment, 20" in the third, etc.

In the present context and in the claims terms like "lower side" or "bottom" and "upper side" or "top" used in connection with the sides of the respective foils will be construed as meaning "skin-facing side" and "opposite the skin-facing side", respectively.

In Fig. 1a a first embodiment of an epidural catheter fixation device is designated the reference numeral 10 in its entirety. The epidural catheter fixation device comprises a basic foil 12 having a first adhesive side and a second non-adhesive side. A major, substantially' rectangular part of the basic foil 12 is designated the reference numeral 14 and is centrally provided with a circular inspection window 30, which is provided by the removal of a circular portion of the basic foil material providing an aperture, which is covered by a transparent plastic foil. The first adhesive side of the basic foil 12 is covered by a release paper 16, which is partly removed in the view illustrated in Fig. 1a.The major part of the basic foil 12 extends through a narrowing into a neck and further continuing into a generally U-shaped foil portion having outwardly pointing uneven branches. The transition from the rectangular major part of the basic foil 12 to the neck is in Fig. 1 a indicated by a phantom line 19.

The adhesive applied to the first adhesive side of the basic foil 12 constitutes a skin friendly adhesive. For fixating the epidural catheter to be fixated by means of the device 10, the generally U-shaped foil portion of the basic foil 12 is covered by an adhesive foil 20 having an adhesive coating exhibiting a stronger bonding capability to the material of the epidural catheter as compared to the bonding capability of the skin friendly adhesive material of the basic foil 12. The foil 20 has, as is illustrated in Fig. 1a, the same shape as the U-shaped foil part of the basic foil 12.

A substantially U-shaped first pad 24 with substantially even branches is adhered to the foil 20. The substantially U-shaped pad 24 defines an outer convex marginal surface constituting a support and contact surface against which a catheter to be fixated by means of the device 10 is positioned as will be described in greater details below. A second pad 22 is adhered to the foil 20 at the extremity of the longer branch of the foil 20 on the same side thereof as the U-shaped pad 24.

In Fig. 1 b, the lower side of the first embodiment of the epidural catheter fixation device 10 according to the present invention is shown disclosing the second non-adhesive side of the basic foil 12 and also the inspection window 30. The bottom view of the epidural catheter fixation device 10 shown in Fig. 1 b further discloses a specific feature of the device as the generally U-shaped foil portion and also the neck and the adjacent part of the major, rectangular part of the basic foil 12 is covered by an adhesive coating 15, which defines a boundary line 17 serving the essential functional feature of defining a line for folding the first adhesive side of the basic foil 12 onto the skin surface part of the person for correctly positioning the inspection window 30 arresting and the first pad 24 as illustrated in Fig. 1d.

In Fig. 1 b, the adhesive coating 15 is disclosed, however, initially, the adhesive coating 15 is, as is illustrated in Fig. 1a, covered by a release paper 26.

Fig. 1 c shows the first embodiment 10 of an epidural catheter fixation device of Fig. 1a in a first step of applying the fixation device onto a skin surface part of a person for fixating an epidural catheter 32 relative to the skin surface part. Initially, the release paper 26 shown in Fig. 1a is removed exposing the adhesive coating 17 of the basic foil 12. The fixation device 10 is then positioned adjacent to the perforation site as the U-shaped first pad 24 is positioned juxtaposed the perforation site allowing the epidural catheter 32 to be bent backwardly and supported in a recess 23 also shown in Fig. 1a provided in the U-shaped pad 24. The epidural catheter 32 perforates the skin perpendicularly to the patient's skin surface part through a perforation site and is bent backwardly relative to the pad 24 and wrapped round the rounded portion of the pad 24 and positioned in contact with the outer convex marginal surface of the pad 24. The epidural catheter 32 is then positioned below the second pad 22 and below the longer extrimetry of the U-shaped first pad 20.

The epidural catheter 32 is then fixated relative to the skin surface part of the person as the adhesive of the adhesive foil 20 is caused to adhere to the epidural catheter 32. The wrapping of the catheter 32 round the U-shaped pad 24 firstly ensures that the catheter 32 does not bend brusquely, in an acute angle, which might lead to catheter constriction and preventing the flow of fluid through the catheter. The wrapping of the catheter round the pad 24, secondly forms a loop in a plane substantially perpendicular to the plane of the catheter perforating the skin surface at the perforation site and pull relief in case the catheter 32 is pulled, squeezed, pressed, jerked or in any other way removed from its resting position on the adhesive foil 20, thereby diminishing the discomfort caused to the patient and the risk that the catheter may be extracted unwillingly from its insertion site.

Thereupon, the first release paper 16 shown in Fig. 1a is removed as is illustrated in Fig. 1c and the basic foil 12 is folded onto itself and also onto the pad 24 supporting the epidural catheter 32 in the above described non-bending and pull relieving manner as the basic foil 12 is bent along the continuous line 17 constituting the margin of the adhesive coating 17. As the basic foil 12 is folded onto the pad 24, the perforation site is also sealed as is illustrated in Fig. 1d illustrating the final positioning of the fixation device 10 relative to the skin surface part of the person. As the basic foil 12 is folded onto the pad 24 and also onto the skin surface part of the person, the adhesive of the first adhesion side of the basic foil 12 adheres to the skin surface part and also adheres to the epidural catheter 32. In the above described first embodiment, the inspection window 30 allows visual inspection of the perforation site and also inspection of the position of the epidural catheter supported by the device. It is to be realized that the epidural catheter is in its bent wrapped round the first pad 24.

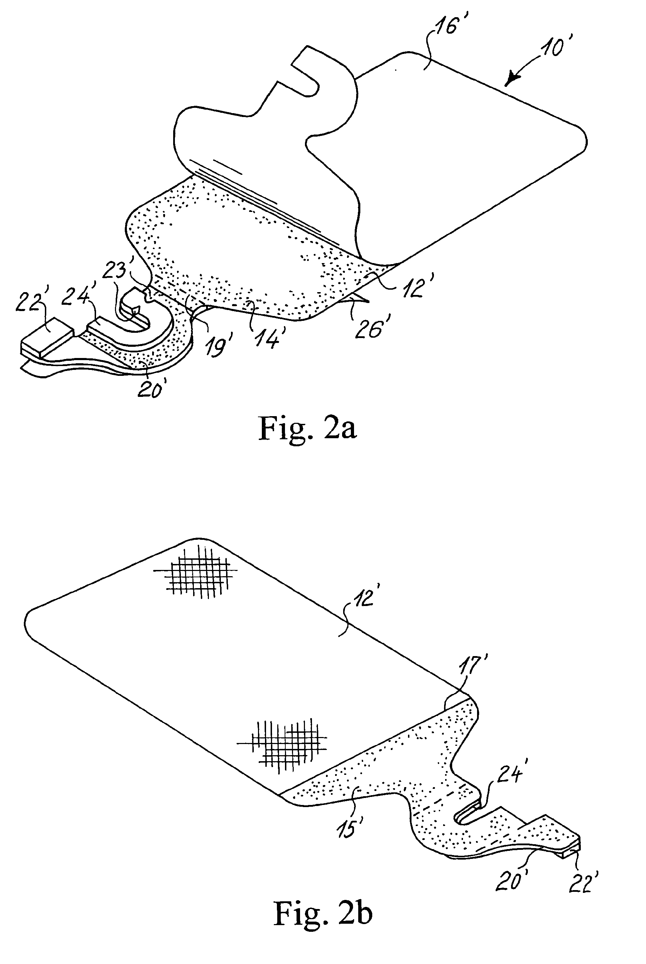

Figs. 2a and 2b shows a second embodiment of an epidural catheter fixation device 10' according to the present invention, which embodiment is designated the reference numeral 10' in its entirety and is very similar in construction to the first embodiment 10 presented in Fig. 1a, but differing in the position and shape of the U-shaped extension of the basic foil 12'. The second embodiment 10' differs from the first embodiment 10 in that the U-shaped pad 20' is shifted 90° relative to the overall orientation of the basic foil 12' as compared to the orientation of the pad 24 in relation to the basic foil 12 of the first embodiment 10. Thus, as is evident from Fig. 1d, the basic foil 12 is applied in an orientation substantially transversly relative to the orientation of the epidural catheter 32, the basic foil 12' is applied lengthwise or longitudinally relative to the overall orientation of the epidural catheter which is to be fixated by means of the device 10'.

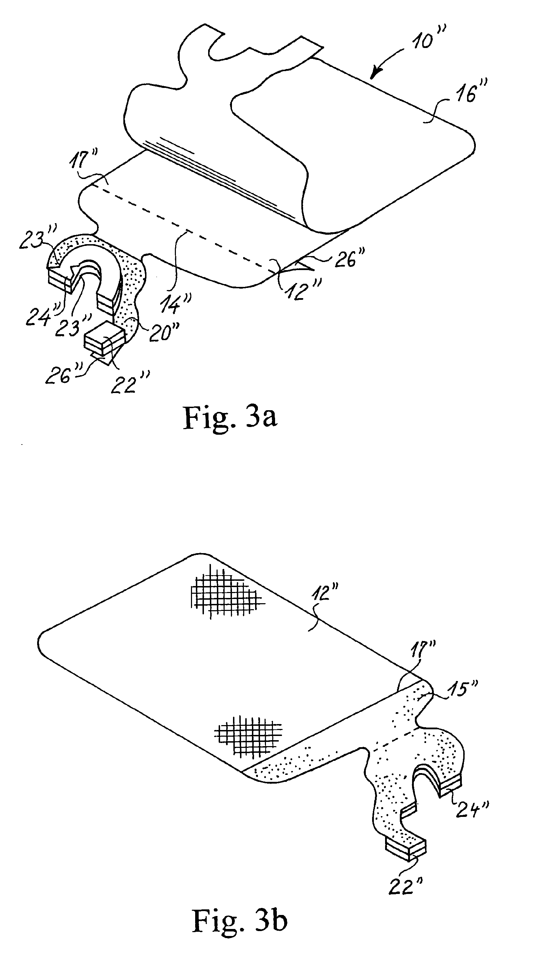

Figs. 3a and 3b show a third embodiment of the epidural catheter fixation device 10" according to the present invention, which embodiment is identical in shape to the first embodiment 10 presented in Fig. 1 a, but its construction is simplified in the respect that this third embodiment does not employ the above-mentioned foil 20, providing a simpler construction. In the third embodiment 10", an adhesive coating 21 is applied to the generally U-shaped foil portion.

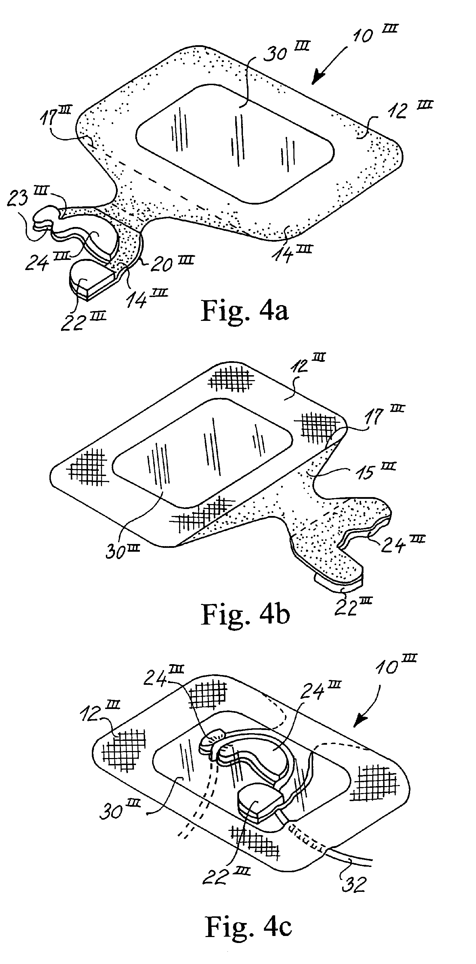

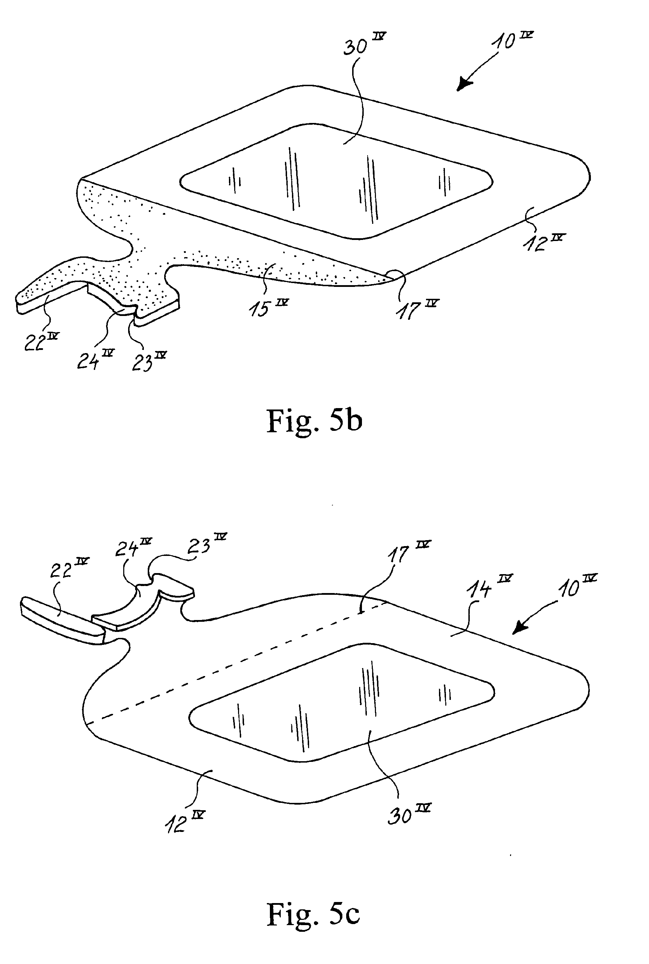

Figs. 4a, 4b and 4c are schematic and perspective views of a fourth embodiment 10"' of the epidural catheter fixation device 10'" according to the present invention. The structure of the fourth embodiment of the epidural catheter fixation device according to the present invention is basically the same as the structure of the first embodiment of the invention, however, in the fourth embodiment 10^{III}, the pad 24^{III} is provided with two grooves 23^{III} for placing and fixating the epidural catheter 32. The configuration of the fourth embodiment is optimized as to limit the requirements of dexterity from the user's part and also the multiple bending of the epidural catheter 32, thus reducing the risk of constriction of the flow through the catheter 32. The epidural catheter 32 is thus placed above the section between the two grooves 23^{III}, then wrapped round the semicircular curvature of the pad 24^{III}, and finally introduced under the second pad 22^{III} which adheres to the patients skin. Then the generally U-shaped part of the basic foil 12^{III} is folded onto itself along the folding'line 17^{III} which marks the margin of the adhesive layer 15^{III}. The finally fixated epidural catheter 10^{III} is seen in Fig. 5c.

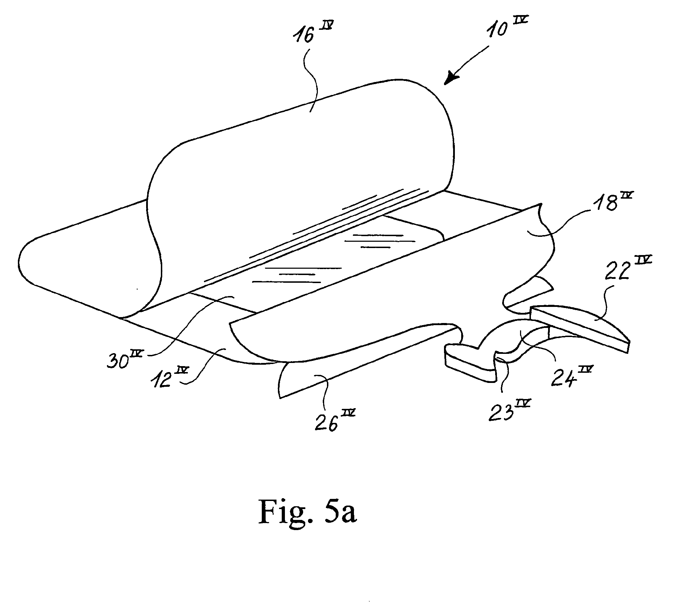

Figs. 5a, 5b and 5c are schematic and perspective views of a fifth and preferred embodiment of the epidural catheter fixation device 10^{IV} according to the present invention.

In the fifth embodiment, the design of the pads 22^{IV} and 24^{IV} has been refined, firstly by providing the pad 22^{IV} as an elongated pad having a substantially straight wall along which the epidural catheter is fixated and having a tapering inlet for guiding the epidural catheter into its intentional position and further providing a constriction for the arresting of the epidural catheter 32 relative to the spacing between the two pads 22^{IV} and 24^{IV}. Secondly, the pads 22^{IV} and 24^{IV} have been configurated so as to provide a pull relief function of the epidural catheter fixation device 10^{IV} at the contsriction between the pads 22^{IV} and 24^{IV}, i.e. at a part of the epidural catheter in which the epidural catheter 32 is fixated after the epidural catheter has been turned along the rear wall of the pad 24^{IV}, consequently, the pull relief established by the constriction between the pads 22^{IV} and 24^{IV} is established after the turning of the epidural catheter.

### Example

The epidural catheter fixation device 10^{IV} shown in Figs: 5a, 5b and 5c was made from the following materials: The basic foil 12^{IV} was made from a non-woven polyamide sheet of approx. 45 g/m². The overall length of the basic foil 12^{IV} was 115 mm and the overall width of the basic foil 12^{IV} was 105 mm. The transparent window 30^{IV} was a semi-permeable polyurethane sheet. The adhesive materials applied to the basic foil 12^{IV} were medical grade acrylic adhesives. The pads 22^{IV} and 24^{IV} were made of polyethylene foam. The adhesives of the fixating device were covered by a siliconized release paper.

Although the present invention has been described above with reference to specific, presently preferred embodiments of the fixation device according to the present invention, the present invention is, however, by no means to be construed as limited to the above embodiments, as numerous modifications, combinations and amendments are readily perceivable by a person having ordinary skill in the art, within the scope of the present invention as defined in the appended claims.

## Claims

1. A fixation device for fixating a catheter relative to a skin surface part of a person, said catheter defining an outer diameter and being introduced into the interior of said person through said skin surface part substantially perpendicularly relative thereto through a perforation site, comprising:
a basic foil of a foil material having opposite first and second surfaces and defining a first end,
a first adhesive coating applied to said first surface of said basic foil,
a first pad to be arranged adjacent to said perforation site and defining a convex marginal surface having a radius of curvature of at least one order of magnitude larger than said outer diameter of said catheter, said marginal surface providing a support surface in relation to said catheter positioned in facial contact therewith for arranging said catheter in a loop and turning said catheter from an orientation substantially perpendicularly relative to said skin surface part to an orientation substantially parallelly with said skin surface part and for preventing said catheter from sliding along said marginal surface, said first pad being connected to and supported by said basic foil at said first surface of said basic foil, and
a second adhesive coating applied to a part of said second surface of said basic foil below said first pad and defining a rectilinear adhesive material boundary line constituting a folding line for folding said basic foil onto said first pad in a specific position relative thereto and covering said first pad, and for arranging said first surface of said basic foil facing towards said first pad for adhering to said skin surface part, for fixating said catheter relative to said first pad and for covering said perforation site.

2. The device according to claim 1, further comprising a second pad connected to and supported by said basic foil and positioned adjacent to said first pad for defining with said first part a constriction having a width slightly smaller than said outer diameter of said catheter for arresting said catheter within said restriction when received therein.

3. The device according to claim 2, said said constriction constituting a tapering constriction for allowing said catheter to slide relative to said transition in the one direction relative to said constriction and preventing said catheter from sliding in the opposite direction.

4. The device according to any of the claims 1-3, said first pad being provided with an adhesive covering at said convex marginal surface.

5. The device according to any of the claims 1-4, said first pad being provided with an adhesive surface covering.

6. The device according to any of the claims 1-5, said basic foil providing a fixation support adjacent said first pad for attaching said catheter to said skin surface part of said person.

7. The device according to any of the claims 1-6, said first pad having two opposed notches.

8. The device according to any of the claims 1-7, said basic foil having a transparent area allowing visual inspection of said perforation site.

9. The device according to any of the claims 1-8, said basic foil being covered by a release paper covering said adhesive material of said first surface of said basic foil.

10. The device according to any of the claims 1-12, said basic foil being of a rectangular configuration, a quadratic configuration, a circular configuration, an elliptic configuration or a combination of the above configurations, and having an extention, at which said first pad be positioned.

11. The device according to any of the claims 1-10, said basic foil being made of a water-permeable material.

12. The device according to any of the claims 1-13, said basic foil being made of a water-impermeable material.

13. The device according to any of the claims 1-12, said adhesive coating being skin friendly adhesive coatings.

14. A catheter system comprising:
a catheter defining an outer diameter and being introduced into the interior of said person through said skin surface part substantially perpendicularly relative thereto through a perforation site, and
a fixation device according to any of the claims 1-13.
